# EUROPÄISCHE PATENTSCHRIFT

(11) **EP 0 934 058 B1**
(45) Veröffentlichungstag und Bekanntmachung des Hinweises auf die Patenterteilung: **20.08.2003**
(21) Anmeldenummer: 97929304.0
(22) Anmeldetag: 27.06.1997
(51) Int. Cl.: A61K 9/16, A61K 9/50, A61K 31/415, A61K 9/28

(54) **STABILE ARZNEIFORM MIT BENZIMIDAZOLDERIVATEN ALS WIRKSTOFF ZUR ORALEN VERABREICHUNG UND VERFAHREN ZU IHRER HERSTELLUNG**
STABLE DRUG FORM FOR ORAL ADMINISTRATION WITH BENZIMIDAZOLE DERIVATIVES AS ACTIVE INGREDIENT AND PROCESS FOR THE PREPARATION THEREOF
FORME STABLE DE MEDICAMENT POUR ADMINISTRATION PAR VOIE ORALE, A BASE DE DERIVES DE BENZIMIDAZOLE COMME PRINCIPE ACTIF, ET SON PROCEDE DE PRODUCTIONN

(30) Priorität: 28.06.1996 DE 19626045
(43) Veröffentlichungstag der Anmeldung: 11.08.1999
(73) Patentinhaber: AstraZeneca AB, 151 85 Södertälje (SE)
(72) Erfinder: HEESE, Gerd-Ulfert, D-81825 München (DE); JÜNGER, Herbert, D-85221 Dachau (DE); LAICHER, Arnim, D-81541 München (DE); LORCK, Claudio, D-81476 München (DE); PROFITLICH, Thomas, D-81929 München (DE); WEISS, Gerd, D-81667 München (DE)
(74) Vertreter: HOFFMANN - EITLE
(86) Internationale Anmeldenummer: EP9703387
(87) Internationale Veröffentlichungsnummer: WO98000114

(56) Entgegenhaltungen:
- EP-A- 0 348 808
- EP-A- 0 496 437
- WO-A-94/02140
- WO-A-95/01783

## Beschreibung

Die vorliegende Erfindung offenbart eine stabile Arzneiform zur oralen Verabreichung, welche als Wirkstoff eines oder mehrere der Benzimidazolderivate Omeprazol, Lansoprazol oder Pantoprazol enthält, sowie ein Verfahren zu ihrer Herstellung.

Aus EP 0 005 129 ist bekannt, daß Omeprazol (5-Methoxy-2-(((4-methoxy-3,5-dimethyl-2-pyridyl)methyl)sulfinyl)-1Hbenzimidazol) als ein potenter Inhibitor bei der Sekretion von Magensäure fungiert. Omeprazol hat sich in der Therapie von Ulcus duodeni, Ulcus ventriculi, Refluxösophagitis und Zollinger-Ellision-Syndrom bewährt. Dabei kommen parenterale und feste perorale Arzneimittel zur Anwendung.

Die folgenden für Omeprazol gemachten Ausführungen gelten in gleicher Weise für Lansoprazol (2-(((3-Methyl-4-(2,2,2-trifluorethoxy)-2-pyridyl)methyl)sulfinyl)-1H-benzimidazol) und Pantoprazol (5-Difluormethoxy-2-((3,4-dimethoxy-2-pyridyl)methylsulfinyl)-1H-benzimidazol).

Die Verabreichung eines Arzneimittels *per os* ist besonders bequem, da sie ohne Aufwand und unangenehmen Begleiterscheinungen vom Patienten selbst praktisch überall und zu jeder Zeit durchgeführt werden kann. Der orale Verabreichungsweg bringt es zwangsläufig mit sich, daß das Arzneimittel zunächst in den Magen gelangt. Omeprazol und dessen Derivate degradieren jedoch sehr schnell im sauren Milieu des Magens zu unwirksamen Verbindungen. In wässriger Lösung hat Omeprazol beispielsweise bei pH-Werten von unter 4 eine Halbwertszeit von weniger als 10 Minuten. Feste perorale Arzneiformen (Tabletten, Pellets, Granulate) von Omeprazol und ähnlichen Wirkstoffen müssen deshalb vollständig gegen Magensaft geschützt werden.

Die Resorption von Omeprazol erfolgt im oberen Duodenum, wobei dieser Wirkstoff einen ausgeprägten First-Pass-Effekt zeigt.

Es muß daher eine möglichst rasche und vollständige Freisetzung des Wirkstoffes aus der Arzneiform nach Passage des Pylorus sichergestellt werden, um ausreichend hohe Bioverfügbarkeit zu gewährleisten.

Omeprazol wird hierzu mit einem Überzug aus enterischen, d.h. magensaftresistenten Materialien versehen, der einerseits im sauren Milieu des Magens (ca. pH 1 bis 3) unlöslich ist, sich aber andererseits im schwach sauren bis schwach alkalischen Bereich des Duodenums (pH >5,5) auflöst. Bekannt ist, die extrem säureempfindliche Wirksubstanz Omeprazol in den Kern einer Pelletformulierung einzubringen, auf welcher eine oder mehrere Überzugsschichten vorgesehen sind.

Als Schichtmaterial wird häufig Eudragit® L100 oder L100-55 benutzt. Eudragit® L100 ist ein Copolymer aus Methacrylsäure und Methylmethacrylat in einem bestimmten Verhältnis und ist in saurem Milieu, z.B. im Magen, unlöslich und bildet somit eine weitgehend undurchlässige Schutzschicht aus. Eudragit® L100-55 ist ein Copolymer aus Methacrylsäure und Ethylacrylat, wobei das Verhältnis der Monomeren so ausgewählt ist, daß es bei einem pH <5,5 unlöslich, bei einem pH darüber aber löslich ist. Der Grund dafür liegt im wesentlichen darin, daß die Carboxylseitengruppen des Polymers im sauren Milieu protoniert vorliegen, und das Polymer damit insgesamt ungeladen ist. Im schwach sauren, neutralen bzw. basischen Milieu, z.B. im Darmbereich, deprotonieren die Carboxylgruppen, wodurch das Polymer negative Ladungen erhält. Es wird wasserlöslich, wobei der Wirkstoff freigesetzt wird.

Eudragit kann jedoch nicht direkt auf den Omeprazol-Kern aufgebracht werden, da die Carboxylgruppen in der Überzugsschicht das Omeprazol degradieren, was auch Probleme bei der Herstellung und bei der Lagerung der Arzneiform mit sich bringt. Schon geringe Mengen an Degradationsprodukten führen zu deutlichen Farbveränderungen und damit Qualitätseinbußen, die unter Umständen keine Verabreichung am Patienten mehr erlauben. Die Lagerungsprobleme werden verschärft, wenn durch Haarrisse und andere Defekte in der Überzugsschicht Feuchtigkeit in den wirkstoffhaltigen Kern eindringt.

Für den Schutz fester peroraler Arzneimittel mit Omeprazol, Lansoprazol oder Pantoprazol als Wirkstoff vor ungünstigen Lagerungsbedingungen und bei der oralen Einnahme vor dem Magensaft eignen sich magensaftresistente Überzüge aus den obengenannten Polymeren, die durch eine inerte Isolierschicht vom wirkstoffhaltigen Kern getrennt werden. Darüber hinaus hat es sich als sinnvoll erwiesen, den wirkstoffhaltigen Kern durch Zusatz einer alkalisch reagierenden Substanz zu stabilisieren. Andererseits muß eine ausreichend schnelle Freisetzung im Darm sichergestellt sein.

### Stand der Technik

DE 1 204 363 beschreibt eine Arzneiform bestehend aus einem Kern mit drei darauf aufgebrachten, unterschiedlichen Schichten. Die erste (innerste) Schicht ist im Magen löslich, im Darm aber unlöslich. Die zweite Schutzschicht ist wasserlöslich (unabhängig vom pH-Wert) und die dritte (äußere) Schutzschicht ist ein magensaftresistenter Überzug. Für Omeprazol ist diese Formulierung jedoch nicht geeignet, weil sie sich nur langsam im Darm auflöst. Eine schnelle Auflösung im Darm ist jedoch für die angestrebte Bioverfügbarkeit wesentlich.

EP 0 247 983 offenbart ein pharmazeutisches Mittel zur oralen Verabreichung, das Omeprazol als wirksamen Bestandteil umfaßt. Das Kernmaterial enthält Omeprazol zusammen mit einer alkalisch reagierenden Verbindung oder einem Omeprazol-Salz, gegebenenfalls zusammen mit einem alkalisch reagierenden Hilfsstoff. Zwischenschichten, die eine Trennschicht zwischen dem alkalisch reagierenden Kern und einer äußeren Schicht aus einem magensaftresistenten Überzug bilden, umfassen wasserlösliche oder darin rasch zerfallende Tablettenträgermittel oder polymere, wasserlösliche, filmbildende Substanzgemische, die gegebenenfalls puffernde, alkalische Verbindungen enthalten und von außen eindringende Protonen abfangen sollen. Abgesehen von seiner Wasserlöslichkeit ist das Schichtmaterial chemisch und physikalisch inert.

Allerdings kann bei Verwendung einer alkalisch puffernden Substanz, wie z.B. Natriumacetat, diese in der Zwischenschicht frei diffundieren und in die äußere magensaftresistente Schicht eindringen. Die damit einhergehende Erhöhung des pH-Wertes kann das Vordringen von Feuchtigkeit durch die enterische Schicht aufgrund der zunehmenden Löslichkeit begünstigen. Dies bedeutet, daß bei Eindringen höherer Konzentrationen von Protonen die Gefahr besteht, daß diese in den Kern gelangen, und dort das Omeprazol zerstören. Letzteres kann insbesondere dann leicht eintreten, wenn die äußere magensaftresistente Schicht aufgrund von Imperfektionen, wie sie sich bei der Herstellung ergeben können, physikalischer Belastung oder durch Alterungserscheinungen bei der Lagerung Risse und Sprünge aufweist.

EP 0 519 144 beschreibt Omeprazol-Pellets bestehend aus einem inerten Pelletkern, der mit dem mikronisierten Wirkstoff beschichtet ist und anschließend mit einer magensaftresistenten Schicht überzogen wird. Zur Beschichtung des Kerns mit Omeprazol werden folgende Hilfstoffe, dispergiert in Wasser, eingesetzt: Hydroxymethylcellulose (HMC), wasserfreie Lactose, L-Hydroxypropylcellulose (L-HPC), Natriumlaurylsulfat, Dinatriumhydrogenphosphatdihydrat. Als magensaftresistenter Überzug wird Hydroxypropylmethylcellulose-phthalat (HPMCP) verwendet. Bei dieser Vorgehensweise ist eine mögliche Reaktion des Omeprazol mit dem Polymer nicht ausgeschlossen, was insbesondere zu einer verschlechterten Lagerungsstabilität führen kann.

EP 0 496 437 umfaßt Pelletkerne bzw. Tabletten, die Omeprazol oder ein Alkalisalz von Omeprazol zusammen mit einer alkalisch reagierenden Verbindung (Puffer) enthalten und mit einer Schicht wasserlöslicher, filmbildender Hilfstoffe, die bevorzugt alkalisch reagieren (Puffer), sowie mit einem magensaftresistenten äußeren Film überzogen sind.

EP 0 237 200 verwendet basische Magnesiumsalze und/oder basische Calciumsalze zur Stabilisierung von Benzimidazol-Derivaten mit Omeprazol als einen typischen Vertreter.

Es wurden demnach zahlreiche Bemühungen zur Herstellung von Omeprazol-Arzneiformen unternommen, die die Verfärbung des Wirkstoffes verhindérn, den chemischen Abbau von Omeprazol weitgehend reduzieren, die Degradation des Wirkstoffes im sauren Magensaft unterbinden, aber gleichzeitig im Milieu des Dünndarms den Wirkstoff möglichst rasch freigeben sollen.

WO-A-94/02140 offenbart eine Arzneiform, die aus einem Kern, einer Zwischenschicht und einer Außenschicht besteht, wobei die Zwischenschicht aus einem wasserlöslichen Polymer besteht.

Aufgabe der vorliegenden Erfindung ist es, eine zur oralen Verabreichung geeignete, gegenüber dem Stand der Technik verbesserte Arzneiform zur Verfügung zu stellen, die als Wirkstoff Omeprazol, Lansoprazol und/oder Pantoprazol, gegebenenfalls in Kombination mit weiteren pharmazeutisch wirksamen Substanzen, enthält, und bei ausgedehnter Lagerung und unter chemisch-physikalischer Belastung eine ausgezeichnete Stabilität besitzt. Insbesondere soll mit der erfindungsgemäßen Arzneiform das Eindringen von saurem Magensaft an Sprüngen, Rissen, Kanten oder jeglichen anderen Imperfektionen der Überzugsschicht in die Kernschicht vermieden und dadurch ein Abbau der säurelabilen Wirkstoffe verhindert werden.

Die erfindungsgemäße Arzneiform gewährleistet eine sehr hohe Arzneimittelsicherheit, die vor allem auch dann gegeben sein soll, wenn sich im Verlauf des Herstellungsverfahrens der Arzneiform sowie bei der Handhabung derselben bzw. deren Verpackungsform durch den Patienten ungünstige Bedingungen ergeben.

Gleichzeitig ist es erforderlich, daß die Arzneiform nach Passage durch den Magen den Wirkstoff im Dünndarm rasch freisetzt. Ferner soll der Arzneiaufbau das Auftreten von Verfärbungen des Wirkstoffes verhindern.

Die vorstehende Aufgabe wird erfindungsgemäß gelöst durch eine stabile Arzneiform zur oralen Verabreichung, welche
(a) einen Kern, der einen Wirkstoff, ausgewählt aus Omeprazol, Lansoprazol und Pantoprazol, zusammen mit üblichen pharmazeutischen Hilfsstoffen enthält,
(b) eine auf den Kern aufgebrachte Zwischenschicht, und
(c) eine magensaftresistente Außenschicht umfaßt, und die sich dadurch auszeichnet, daß
   in (b) eine reaktive Zwischenschicht aus einem mit Alkalien teilneutralisierten magensaftresistenten Polymer-Schichtmaterial mit Kationenaustauscherkapazität vorliegt.

Gegenstand der Erfindung ist ferner ein Verfahren zur Herstellung der vorstehend genannten Arzneiform, wonach man
(a) als Kern Formling bildet, der einen Wirkstoff, ausgewählt aus Omeprazol, Lansoprazol und Pantoprazol, zusammen mit üblichen pharmazeutischen Hilfsstoffen enthält,
(b) auf den Formling eine Zwischenschicht aufbringt, und
(c) den überzogenen Formling mit einer magensaftresistenten Schicht befilmt,
und das Verfahren dadurch gekennzeichnet ist, daß man in (b) eine reaktive Zwischenschicht aus einem mit Alkalien teilneutralisierten magensaftresistenten Polymer-Schichtmaterial mit Kationenaustauscherkapazität aufbringt.

Bevorzugte Ausführungsformen der Erfindung sind in den abhängigen Ansprüchen wiedergegeben.

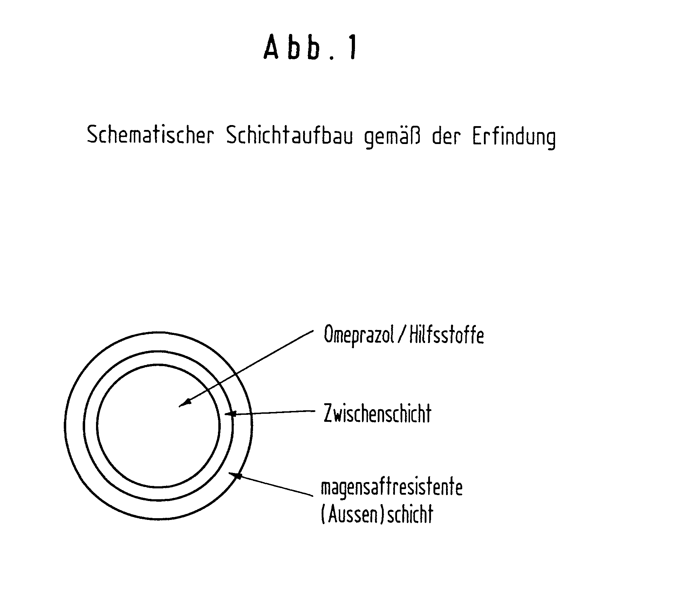

Der Schichtaufbau der erfindungsgemäßen Arzneiform ist schematisch in Abbildung 1 wiedergegeben.

Der Kern der erfindungsgemäßen Arzneiform umfaßt den Wirkstoff Omeprazol, Lansoprazol oder Pantoprazol einzeln oder Kombinationen davon zusammen mit üblichen Hilfssubstanzen. Für die Stabilität der erfindungsgemäßen Arzneiform ist es nicht erforderlich und auch nicht bevorzugt, daß der Kern aus dem Wirkstoff zusammen mit einer alkalisch reagierenden Verbindung formuliert wird. Es ist auch nicht notwendig, daß ein alkalisches Salz des Wirkstoffes eingesetzt wird.

Als pharmazeutische Hilfsstoffe für den Kern eignen sich insbesondere Füllstoffe, wie Mannit, Hydroxypropylcellulose, mikrokristalline Cellulose und wasserfreie Lactose. Darüber hinaus hat sich gezeigt, daß bei Verwendung einer speziellen Kombination von Mannit und Hydroxypropylcellulose als nicht alkalisierende Hilfsstoffe im Kern vorteilhafte Stabilitätseffekte erzielt werden können.

Der Kern kann auch Netzmittel umfassen, die in geeigneter Weise ausgewählt sind aus Natriumlaurylsulfat, Sorbitanfettsäureester und Polyoxyethylensorbitan-Fettsäureester.

Der Kern der erfindungsgemäßen Arzneiform kann als Formling ausgebildet sein. Bevorzugte Formlinge sind Pelletkerne, Tabletten, Mikrotabletten oder Granulate.

Die Formlinge sind mit einer Zwischenschicht überzogen. Diese Zwischenschicht weist bevorzugt eine Schichtdicke von etwa 5 bis 30µm auf. Sie bildet sowohl eine mechanische als auch chemische Barriere zum Kern hin. Dabei ist es erforderlich, daß diese Zwischenschicht als intakter Film vorliegt. Das Polymer der Zwischenschicht macht etwa 3 bis 5 Gew.-% des Kerngewichts aus.

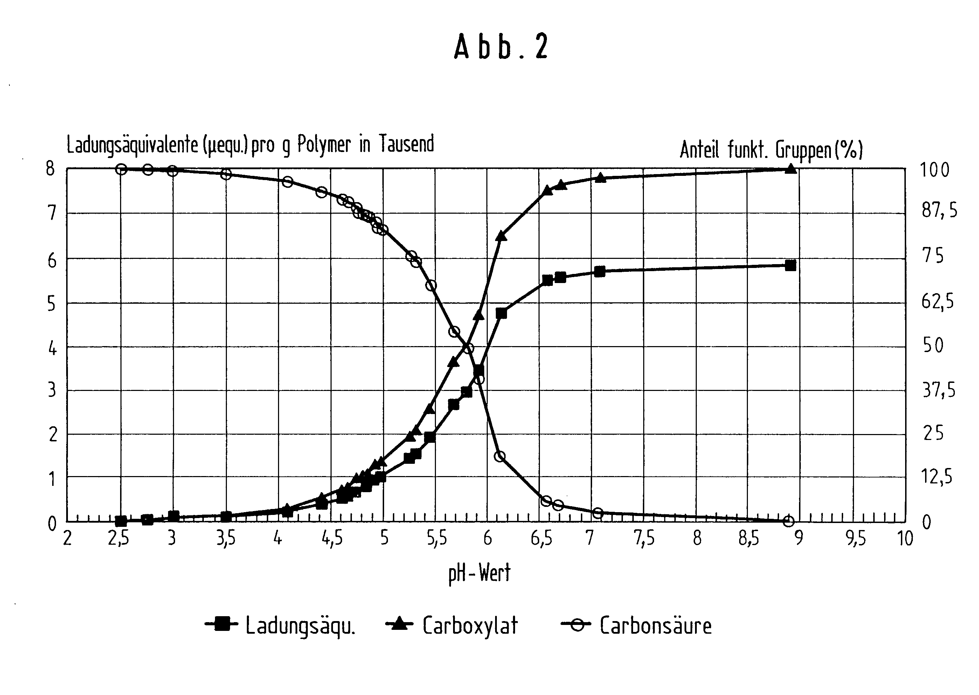

Die Zwischenschicht umfaßt ein magensaftresistentes Polymer-Schichtmaterial, das mit Alkalien auf einen pH-Bereich von 5,5 bis 7,0, bevorzugt 5,5 bis 6,5 eingestellt wurde. Bei diesen pH-Werten sind noch nicht alle Protonen der Säurefunktionen des Polymermaterials ausgetauscht, das Material ist lediglich teilneutralisiert. Wie aus Abbildung 2 hervorgeht, liegen im Falle von Eudragit bei einem pH von 5,5 weniger als 40 % der Carboxylfunktionen neutralisiert vor. Trotzdem ist eine Kombination von auf pH 5,5 teilneutralisiertem Eudragit mit Omeprazol unerwartet auch unter verschärften Lagerungsbedingungen noch stabil (siehe Beispiel 2). Bei pH 7,0 sind ca. 97 % der Carboxylfunktionen des Eudragits neutralisiert (siehe Abbildung 2).

Unter Alkalien versteht man Substanzen, deren Lösung mit Wasser alkalische Reaktionen zeigen (Römpps Chemie Lexikon, 8.Auflage, 1979). In erster Linie gehören hierzu die Hydroxide der Alkalimetalle, insbesondere Natrium und Kalium, aber auch die Hydroxide der Erdalkalimetalle. Erfindungsgemäß bevorzugt sind die Hydroxide der Alkalimetalle, insbesondere Natriumhydroxid.

Bei der Teilneutralisierung werden die Protonen der an den Polymerketten des Schichtmaterials anhängenden Säurefunktionen, z.B. Carboxylgruppen, teilweise durch z.B. Alkalimetallionen als Gegenionen ersetzt. Das derart modifizierte Polymer-Schichtmaterial ist in Gegenwart von Protonen nicht mehr physikalisch-chemisch inert, sondern reaktiv, da es nunmehr Kationenaustauscherkapazität besitzt. Dies bedeutet, daß bei Eindringen von Feuchtigkeit und insbesondere saurem Magensaft an Rissen, Spalten, Kanten oder anderen Imperfektionen durch die äußere Schicht der erfindungsgemäßen Arzneiform die eindringenden Protonen abgefangen und durch unschädliche Alkalimetallionen ausgetauscht werden. Ein weiterer Aspekt des reaktiven Prinzips des Zwischenschichtmaterials zeigt sich darin, daß sich die Zwischenschicht an diesen Stellen zu einer magensaftresistenten Barriere umwandelt, sie besitzt gewissermaßen einen "*Self-Repair*-Mechanismus". Praktische Versuche haben gezeigt, daß sich bei Kontakt der Zwischenschicht mit einem sauren Medium eine gelartige Substanz ausbildet, welche nicht nur Protonen abfängt, sondern auch eine flexible mechanische Barriere ausbildet, die das weitere Eindringen von Feuchtigkeit bzw. saurem Medium verhindert. Die Teilneutralisierung des Polymermaterials für die reaktive Zwischenschicht auf einen pH-Bereich von 5,5 bis 6,5 ist insbesondere deswegen bevorzugt, weil sich bereits eine magensaftresistente Barriere ausbildet, wenn nur wenige Protonen durch die äußere Schicht eindringen, andererseits der Omeprazolkern noch ausreichend stabil ist.

Hierdurch wird auch ein deutlich verbessertes Stabilitätsverhalten der beanspruchten Arzneiform bei ausgedehnter Lagerung und unter chemisch-physikalischer Belastung erreicht.

Puffernde bzw. alkalisierende Zusätze in der Zwischenschicht, wie z.B. in EP 0 247 983 vorgeschlagen, sind nicht mehr notwendig und können vielmehr schädlich sein, da sie die Löslichkeit der Zwischenschicht erhöhen und damit deren Schutzfunktion mindern. Dies läuft dem erfindungsgemäßen "*Self-Repair*-Mechanismus" geradezu entgegen; je mehr basische Äquivalente in der Zwischenschicht vorhanden sind, desto mehr Protonen müssen von außen eindringen, damit der *"Self-Repair*-Mechanismus" der reaktiven Schicht schnell zum Tragen kommt.

Als bevorzugte Substanzen für die Zwischenschicht eignen sich die von Röhm Pharma, Deutschland, hergestellten Polymermaterialien Eudragit® L100-55, Eudragit® L100, sowie Hydroxypropylmethylcellulosephthalat (HPMCP) und Celluloseacetatphthalat (CAP), die, wie oben beschrieben, vor der Anwendung als Zwischenschicht, also vor dem Aufsprühen derselben, mit Alkalien teilneutralisiert werden. Besonders bevorzugt ist das als kommerzielles Handelsprodukt weltweit erhältliche Eudragit® L100-55.

Die Zwischenschicht kann übliche Zusätze, z.B. einen Weichmacher enthalten. Bevorzugt eignen sich hierfür Triethylcitrat, Acetyltriethylcitrat, acetylierte Monoglyceride, Propylenglykol, Polyethylenglykole.

Die überzogenen Formlinge, d.h. der Kern und die Zwischenschicht, werden dann zur Herstellung der erfindungsgemäßen Arzneiform mit einer Außenschicht überzogen.

Diese Außenschicht stellt eine übliche enterische, magensaftresistente Schicht dar. Als Materialien eignen sich hierfür handelsübliche, wässrige Polymerdispersionen, wie Polymethacrylate, z.B. Eudragit® L100-55 (Röhm Pharma), und Coating CE 5142 (BASF). Außerdem können zur Bildung der magensaftresistenten Schicht auch Polymere verwendet werden, die in organischen Lösungsmitteln löslich sind. Als geeignete Stoffe sind z.B. Phthalate (Celluloseacetatphthalat, Hydroxypropylmethyl-cellulosephthalat) zu nennen. Außerdem kann die Außenschicht der erfindungsgemäßen Arzneiform Antihaftmittel, Dispergierungsmittel, Pigmente und Farbstoffe enthalten. Ein geeignetes Antihaftmittel ist beispielsweise Talkum.

Es wurde überraschenderweise festgestellt, daß die erfindungsgemäße Kombination aus enterischer äußerer Schicht und reaktiver Zwischenschicht gegenüber konventionellen Arzneimittelformen mit inerter Zwischenschicht ein beschleunigtes Auflösungsverhalten im künstlichen Darmsaft (pH ca. 5,8) zeigt. Dieser Effekt läßt nicht nur eine sehr rasche Freisetzung des Wirkstoffes im schwach sauren bis schwach alkalischen Milieu des Dünndarms und damit eine ausgezeichnete Bioverfügbarkeit erwarten, sondern ermöglicht auch eine verbesserte Arzneimittelsicherheit, da die enterische Außenschicht verstärkt werden kann, ohne daß eine gewünschte rasche Freisetzung verzögert wird. Hierdurch kann nicht nur die Magensaftresistenz, sondern auch die Arneimittelstabilität, insbesondere bei ungünstigen Lagerungsbedingungen, weiter verbessert werden. Die Dicke der magensaftresistenten Außenschicht der erfindungsgemäßen Arzneiform beträgt daher 20 bis 60µm (ca. 10 bis 50 Gew.-% bezogen auf den Kern), bevorzugt 30 bis 60µm.

In einer vorteilhaften Ausführungsform der Erfindung besteht die reaktive Zwischenschicht aus auf einen pH-Wert von 5,5 bis 7,0, bevorzugt 5,5 bis 6,5, teilneutralisiertem Eudragit® L100-55, und die äußere Schicht aus handelsüblichem Eudragit® L100-55 (pH ca. 2 bis 3). Der pH-Übergang zwischen äußerer Schicht und Zwischenschicht muß nicht unbedingt sprunghaft sein, sondern kann auch als Gradient ausgebildet sein. Dies kann erreicht werden, wenn von innen nach außen mehrere dünne Eudragit-Schichten aufgebracht werden, die jeweils auf einen abnehmenden pH-Wert teilneutralisiert wurden.

Sowohl die reaktive Zwischenschicht als auch die magensaftresistente Außenschicht kann als Vielzahl von Einzelschichten ausgebildet sein.

Der pH-Übergang an der Grenze von der magensaftresistenten Außenschicht zur reaktiven Zwischenschicht kann als Gradient ausgebildet sein.

Die vorliegende Erfindung umfaßt ferner ein Verfahren zur Herstellung einer stabilen Arzneiform zur oralen Verabreichung, welche Omeprazol, Lansoprazol und/oder Pantoprazol als Wirkstoff enthält.

Gemäß dem erfindungsgemäßen Verfahren werden der Wirkstoff und Hilfsstoffe, wie Mannit, Hydroxypropylcellulose und Natriumlaurylsulfat zusammen mit einem geeigneten Lösungsmittel, bevorzugt Isopropanol, angefeuchtet, granuliert und zu den gewünschten Formlingen (z.B. Pellets, Granulat, Tabletten) nach üblichen Verfahren verarbeitet. Die Formlinge werden anschließend mit einer wässrigen Dispersion, bestehend aus einer mit Alkalien auf einen pH-Wert von ca. 5,5 bis ca. 7,0 teilneutralisierten magensaftresistenten Substanz, bevorzugt Eudragit® L100-55, sowie einem Antihaftmittel und/oder Weichmacher, wie Talkum und Triethylcitrat, z.B. in einer Wirbelschichtapparatur unter Bildung der Zwischenschicht mit Kationenaustauscheraktivität befilmt. Eine dem Eudragit® L100-55 entsprechende Qualität ist auch als fertige Suspension unter der Bezeichnung Eudragit® L30D-55 im Handel erhältlich. Im Anschluß daran erfolgt die Beschichtung mit einer magensaftresistenten Substanz (z.B. Eudragit® L100-55), Talkum und einem Weichmacher (wie z.B. Triethylcitrat) zur Ausbildung der enterischen Außenschicht der erfindungsgemäßen Arzneiform.

Bevorzugt ist die Herstellung von Pellets, die in einer für eine gewünschte Wirkstoffdosis genügenden Menge in Gelatinekapseln gefüllt werden.

Die so hergestellte Kapselformulierung kann neben den die genannten Benzimidazolverbindungen enthaltenden Pellets noch andere Wirkstoffe enthalten. Bevorzugt ist eine Kombination von Diclofenac- und Omeprazol-haltigen Pellets. Die Diclofenac-haltigen Pellets sind bevorzugt nach dem erfindungsgemäßen Verfahren hergestellt, d.h. sie enthalten ebenfalls eine reaktive Zwischenschicht. Sie können aber auch nach bekannten Verfahren, wie z.B. in EP 0 348 808 offenbart, hergestellt werden. In einer weiteren Ausführungsform liegen die Diclofenac-haltigen Pellets als Mischung magensaftresistent umhüllter Pellets und retardiert durchlässiger Pellets vor, die erst in tieferen Darmabschnitten freigesetzt werden.

Kombinationen von nicht-steroidalen Entzündungshemmern und Schmerzmitteln sind an sich bekannt. So nennt EP 0 527 887 beispielsweise die Kombination von Diclofenac (o-(2,6-Dichloranilino)phenylessigsäure), einem hochwirksamen NSAID (Non-Steroidal-Anti-Inflammatory-Drug), mit Misoprostol, die unter der Marke Arthrotec®, Heumann Pharma GmbH, Deutschland, zur Behandlung von schmerzhaften Entzündungserkrankungen eingesetzt wird. Das Prostaglandin-Derivat Misoprostol dient hierbei zur Prävention von NSAID-assoziierten Ulcuserkrankungen.

Die feste Kombination von Diclofenac und Omeprazol hat bei der Langzeitbehandlung von Schmerz/Entzündungen eine Reihe von Vorteilen. So hält eine Kombination von Diclofenac mit Omeprazol die Ulcusrate bei Patienten, die ein hohes Risiko für die Entstehung gastrointestinaler Ulzera aufweisen und die gleichzeitig der Behandlung mit einem NSAID bedürfen, niedrig (Ulcus-Prävention). Ferner erzielt diese Kombination hohe Ulcus-Abheilraten in Verbindung mit ausreichender Schmerzlinderung (Therapie). Aufgrund der guten Wirksamkeit und der guten Verträglichkeit der Kombinationspartner in Verbindung mit einer Verabreichung von nur einmal täglich läßt sich die Patienten-Compliance erheblich steigern.

Die zur unmittelbaren Verabreichung *per os* geeignete Kapselformulierung enthält als Einheitsdosis 25 bis 200 mg, vorzugsweise 75 bis 150 mg Diclofenac und 10 bis 40 mg, vorzugsweise 10 oder 20 mg Omeprazol in erfindungsgemäßen Pellets.

Die Vorteile der erfindungsgemäßen Arzneiform gegenüber Omeprazol- und anderen Benzimidazol-haltigen Arzneiformen des Standes der Technik bestehen insbesondere darin, daß an Imperfektionen der Außenschicht, an denen bei Lagerung Feuchtigkeit oder nach peroraler Verabreichung saurer Magensaft in die Kernschicht vorzudringen vermag, die reaktive Zwischenschicht die Protonen nicht nur abfängt, sondern darüber hinaus in ein magensaftresistentes Schichtmaterial zurückverwandelt wird. Durch diesen "*Self-Repair*-Mechanismus" bildet sich eine gelartige Schicht aus, die das Eindringen von Feuchtigkeit und Säure in den Kern der Arzneiform zu verhindern vermag. Für den Fall, das kein Magensaft eindringt, bleibt diese Zwischenschicht löslich. Unerwarteterweise zeigt die Kombination aus enterischer Außenschicht und reaktiver Zwischenschicht darüber hinaus ein verbessertes Auflösungsverhalten im künstlichen Darmsaft, was auf ein entsprechend gutes Auflösungsverhalten im Dünndarm schließen läßt.

Die Erfindung wird anhand der nachfolgenden Beispiele näher erläutert.

### Beispiel 1

*In vitro* Versuche zur chemisch/physikalischen Stabilität der erfindungsgemäßen Arzneiform: Verreibungen von Omeprazol und Zwischenschichtmaterial

Es wurden Lagerungsversuche mit Verreibungen von Omeprazol und verschieden behandelten Zwischenschichtmaterialien über 32 Tage bei 40°C und 75% relativer Feuchte (rF) durchgeführt. Anschließend wurde mit HPLC untersucht, in welchem Maße der Wirkstoff Omeprazol (Restgehalt in Gew.-%) stabil blieb, zu welchem Prozentsatz Degradationsprodukt entstanden (Flächen-% aus Reinheitschromatogramm) und in welchem Maße sich Verfärbungen einstellten. Dabei wurde Omeprazol mit einem nicht vorbehandelten, zur Ausbildung von magensaftresistenten Überzügen verwendeten enterischen Schichtmaterial (HPMCP, Charge 1 a, und Eudragit® L100-55, Charge 1 b, pH 2-3) und mit erfindungsgemäß vorbehandeltem enterischen Schichtmaterial (Eudragit® L100-55) verrieben und offen unter den angegebenen Bedingungen in Petrischalen gelagert. Das vorbehandelte Eudragit® L100-55 war zuvor mit Natriumhydroxid auf pH 5,5 (Charge 1 c) und pH 7,0 (Charge 1 d) teilneutralisiert worden.

Die Ergebnisse sind in Tabelle 1 wiedergegeben. Die angegebenen Werte entsprechen dem Mittel von zwei Probenaufbereitungen. Die Verfärbungen sind als Farbwerte nach dem "Taschenlexikon der Farben", A.Kornerup und J.H.Wauscher, Muster-Schmidt-Verlag, Zürich, Göttingen, 3.Aufl, 1981, wiedergegeben.

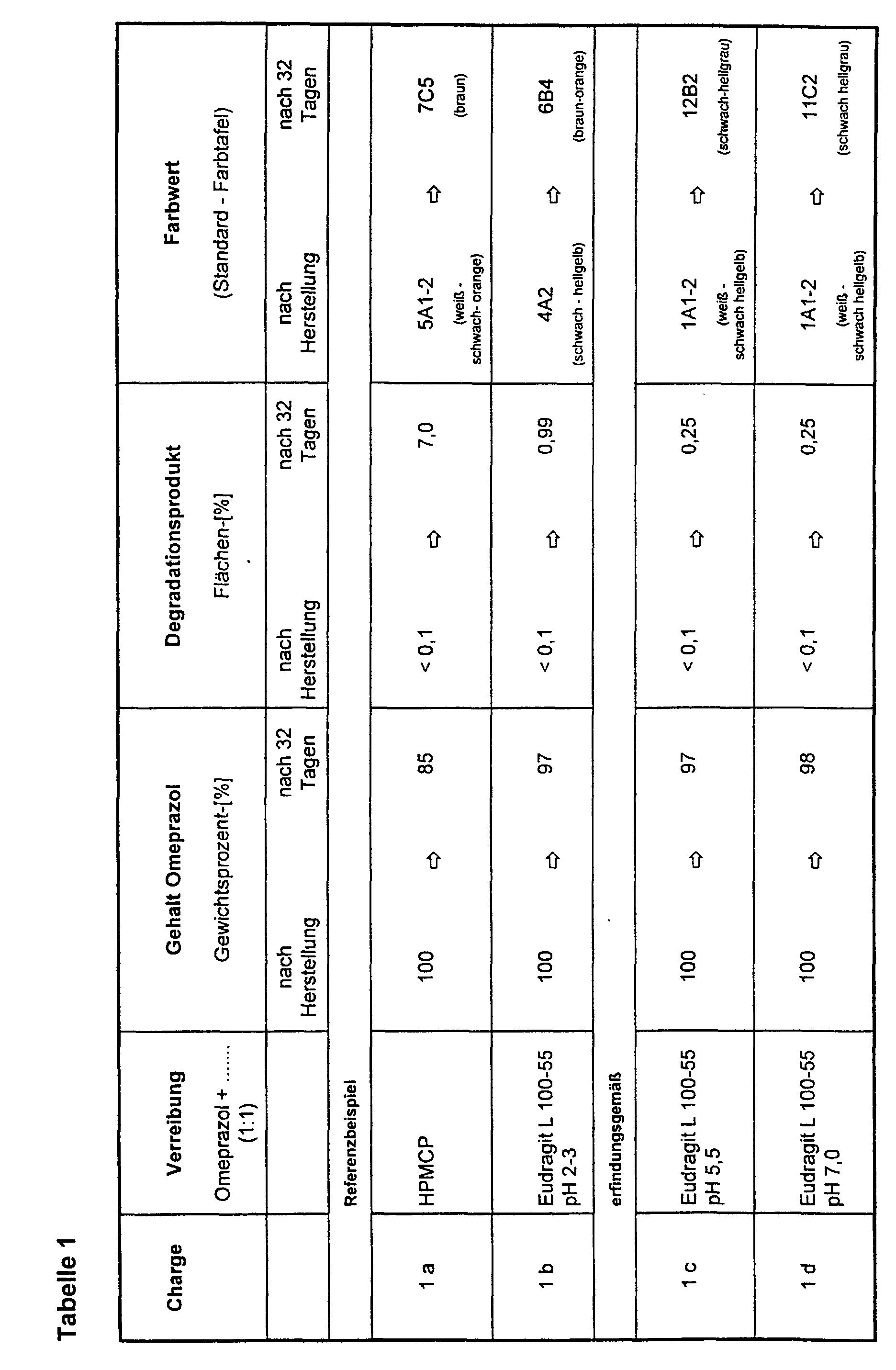

Aus der Spalte "Gehalt Omeprazol" ist zu entnehmen, daß bei Verwendung von teilneutralisiertem magensaftresistenten Polymermaterial gemäß der Erfindung der Wirkstoff wesentlich stabiler bleibt als in einer Verreibung mit einer üblichen enterischen Substanz, die zu 100% freie Carboxylgruppen besitzt. So werden nach 32 Tagen Lagerung unter den genannten Bedingungen erfindungsgemäß nur 2 bzw. 3% des Wirkstoffes Omeprazol abgebaut. Dagegen ist bei Verwendung des im Stand der Technik üblichen enterischen Schichtmaterials HPMCP bei den vorliegenden Verreibungsversuchen nach 32 Tagen ein Omeprazolabbau bis zu 15 Gew.-% festzustellen. Allerdings zeigt sich auch bei Einsatz von nicht neutralisiertem Eudragit noch kein deutlicher Omeprazolabbau (3 Gew.-%).

Dafür ergeben sich beim Vergleich des Gehaltes eines im HPLC-Chromatogramm auftretenden Omeprazol-Degradationsproduktes bei Verwendung von erfindungsgemäßem teilneutralisierten Eudragit (pH 5.5 und 7.0) mit handelsüblichem Eudragit (pH 2 bis 3) deutliche Unterschiede (siehe Spalte "Degradationsprodukt"). So wird gemäß der Erfindung nach 32 Tagen kaum Degradationsprodukt (0,25 Flächen-% in beiden Chargen) festgestellt, während in Gegenwart von handelsüblichen Eudragit (pH 2 bis 3) ca. 0,99 Flächen-%, und in Gegenwart eines üblichen Schichtmaterials (HPMCP) sogar ca. 7% Degradationsprodukt vorliegen. Dieses Ergebnis wird durch den Farbvergleich bestätigt (siehe Spalte "Farbwert"). Weder das braune Produkt der Charge 1 a noch das braun-orange Produkt der Charge 1 b sind verkaufsfähig. Das erfindungsgemäß behandelten Produkte (Chargen 1 c und d) zeigen dagegen eine wesentlich geringere Farbveränderung.

Die vorstehenden Versuche belegen, daß in Gegenwart von hoher Feuchte und erhöhter Temperatur (verschärfter Stabilitätstest) das erfindungsgemäß teilneutralisierte Schichtmaterial auch im verriebenen Zustand schützend auf den Wirkstoff Omeprazol wirkt. Dagegen zeigt übliches enterisches Schichtmaterial, welches zu 100% freie COOH-Gruppen aufweist, nicht nur keine solche Schutzwirkung, sondern verursacht eine deutliche Degradation des Wirkstoffs.

### Beispiel 2

### Stabilität von Pelletformulierungen

In einer weiteren Versuchsreihe wurde die Arzneiform gemäß der Erfindung mit der des Standes der Technik (EP 0 247 983) verglichen. Hierzu wurden verschiedene Pelletchargen hergestellt, die einen dreischichtigen Aufbau zeigen:
- Kern, mit dem Wirkstoff Omeprazol, einmal in Gegenwart einer alkalisch puffernden Substanz (Na₂HPO₄, gemäß Stand der Technik) und einmal ohne alkalisch puffernde Substanz (erfindungsgemäß)
- Zwischenschicht, entweder erfindungsgemäß aus einem mit Alkalien auf pH 6,0 bzw. 7,0 teilneutralisierten enterischen Schichtmaterial, oder inertes Schichtmaterial, das gemäß Stand der Technik als puffernde Substanz Natriumacetat enthält. Das Referenzbeispiel enhält nicht neutralisiertes enterisches Schichtmaterial und Natriumacetat als puffernde Substanz.
- Außenschicht aus Eudragit® L100-55

Außerdem wurde bei einer Versuchsreihe eine Arzneiform getestet, in welcher die Zwischenschicht weggelassen wurde.

Die jeweiligen Pelletchargen wurden bei 40°C und 75% relativer Feuchte (rF) offen in einer Petrischale eine Woche und 20 Tage gelagert. Anschließend wurden der Omeprazolgehalt bzw. das Auftreten von Degradationsprodukt mit HPLC bestimmt. Die in Tabelle 2 zusammengestellten Werte stellen das Mittel von 3 Probenaufbereitungen dar.

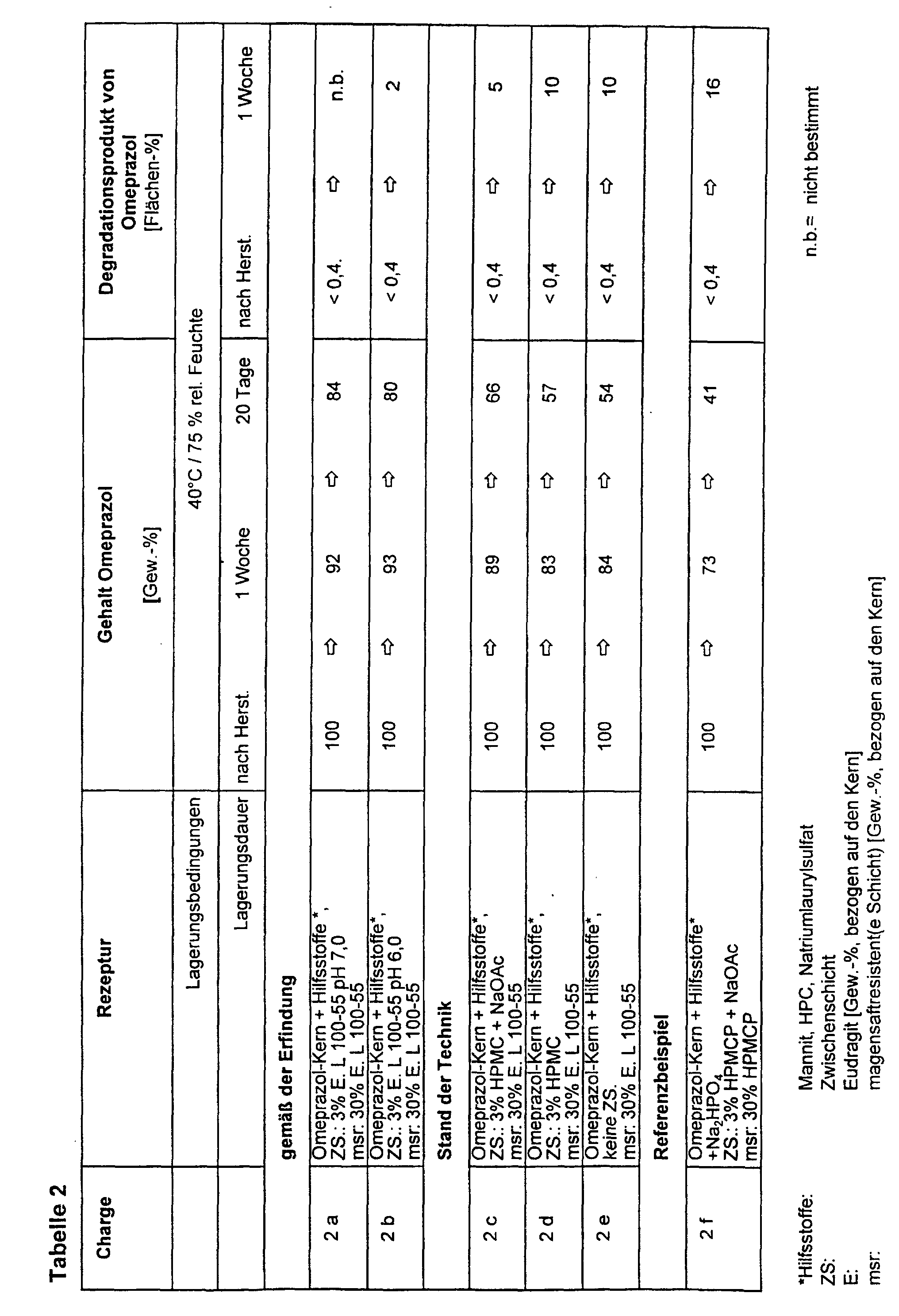

Im Vergleich zum Stand der Technik erhält man erfindungsgemäß eine deutlich stabilere Darreichungsform. Die erfindungsgemäßen Pellets weisen unter den beschriebenen verschärften Lagerbedingungen nach 1 Woche noch 93 Gew.-%, nach 20 Tagen 80 Gew.-% des Wirkstoffes Omeprazol in intakter Form auf. Selbst nach 4-wöchiger Lagerung wurde erfindungsgemäß noch ein Omeprazol-Gehalt von 67 Gew.-% festgestellt (nicht in Tabelle dargestellt. Demgegenüber beträgt der Omeprazol-Gehalt bei nicht erfindungsgemäßen Arzneiformen, das heißt solchen
- mit einer Zwischenschicht aus HPMC und NaOAc
- mit einer Zwischenschicht aus HPMC
- ohne eine Zwischenschicht
- mit einer Zwischenschicht aus HPMCP und NaOAc
nach 20 Tagen lediglich 66, 57, 54 und 41 Gew.-%.

### Beispiel 3

### "Self-Repair-Mechanismus" der reaktiven Zwischenschicht

Verglichen wurden Pellets mit folgendem Aufbau:
- ohne Zwischenschicht (sogenannte Pelletkerne)
- mit der erfindungsgemäßen reaktiven Zwischenschicht
- mit einer inerten Zwischenschicht aus HPMC (Referenzbeispiel)

Zur besseren Beurteilung des Self-Repair-Mechanismus wurden die Pellets nicht mit dem äußeren enterischen Überzug versehen. Alle Pellettypen wurden in künstlichem Magensaft (pH 1,2) in einem Freisetzungsmodell der Europäischen Pharmakopoe (Basket) getestet. Die Zwischenschicht wurde auf pH 7,0, der oberen Grenze des bevorzugten Bereichs, teilneutralisiert.

Die Ergebnisse (ohne Pelletkerne ohne Zwischenschicht) sind in der folgenden Tabelle 3 zusammengefaßt:

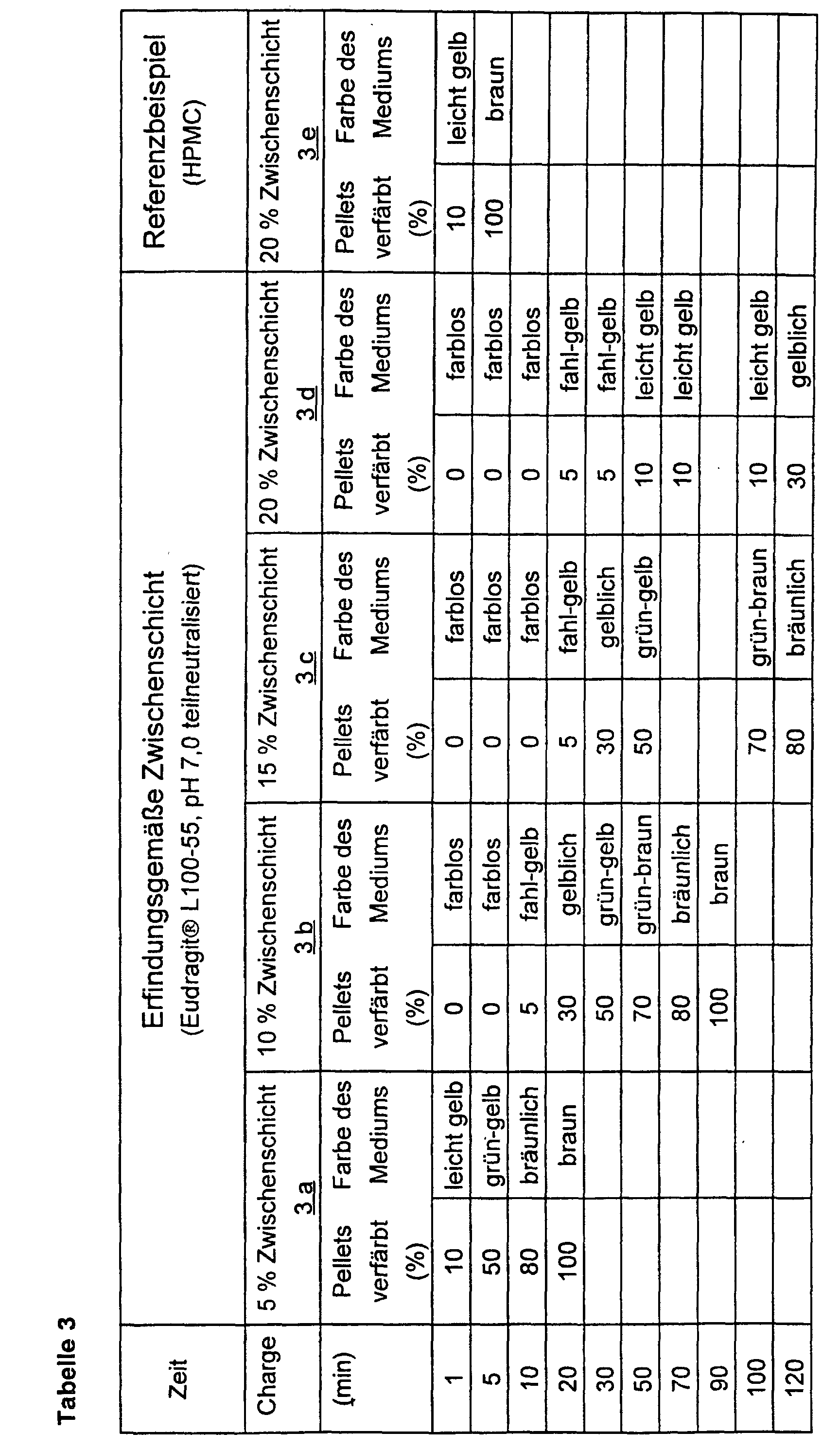

Nach diesen Ergebnissen lösen sich Pellets ohne Zwischenschicht (als Vergleich)
innerhalb von 2 Minuten vollständig auf. Das Freisetzungsmedium weist eine stark braune Verfärbung auf.

Pellets mit der erfindungsgemäßen reaktiven Zwischenschicht bleiben dagegen in Abhängigkeit von der Schichtdicke (5 bis 20 Gew.-%, bezogen auf den Kern) der Zwischenschicht bis maximal 120 Minuten intakt. Das Freisetzungsmedium weist nur eine geringfügige Verfärbung auf.

Pellets mit einer im Stand der Technik üblichen inerten Zwischenschicht (Referenzbeispiel) lösen sich auch bei maximaler Schichtdicke der Zwischenschicht innerhalb von 5 Minuten vollständig auf. Das Freisetzungsmedium weist eine stark braune Verfärbung auf.

Diese Experimente belegen einen meßbaren Schutzmechanismus bei Pellets mit der erfindungsgemäßen Zwischenschicht im Gegensatz zu Pellets mit einer Zwischenschicht nach dem Stand der Technik. Dieser Schutzmechanismus bewirkt die reaktive Umwandlung der Zwischenschicht in eine magensaftresistente Schicht im magensaftsauren Medium. Dies wird umso schneller erfolgen, je näher der pH-Wert der teilneutralisierten Zwischenschicht bei 5,5 liegt.

Aus sämtlichen Untersuchungen in den Beispielen 1 bis 3 geht somit eindeutig hervor, daß gemäß der Erfindung eine Arzneiform mit einer überraschend verbesserten Stabilität im Vergleich zu der des Standes der Technik erhalten wird. Dieses Stabilitätsverhalten zeigt sich insbesondere bei erhöhter Temperatur und Feuchte (Verreibungsversuche), aber auch unter verschärften Lagerbedingungen von 40°C und 75% rF an Pellets.

### Beispiel 4

### Freisetzungsverhalten verschiedener Pelletrezepturen

Wesentlich für eine gute Bioverfügbarkeit des Wirkstoffes ist seine möglichst rasche Freisetzung im oberen Dünndarmbereich, dh. in einem schwach sauren/neutralen Milieu. Zur Überprüfung des Freisetzungsverhaltens wurden Pellets mit verschiedenen Rezepturen in ein wässriges Medium mit einem pH-Wert von 5,8 als *in vitro* Modell für den oberen Dünndarm (künstlichter Darmsaft) eingebracht und das unter Rühren in die Umgebung freigesetzte Omeprazol mit HPLC in Abhängigkeit von der Zeit bestimmt (analog Pharmakopoe).

Die untersuchten Pelletrezepturen und die Freisetzungsergebnisse sind in der Tabelle 4 wiedergegeben:

**Tabelle 4**

| **Charge** | **Pelletrezeptur** | **Freigesetztes Omeprazol [%]** | | |
|---|---|---|---|---|
| | Freisetzungsdauer | 30 min | 45 min | 60 min |

| **gemäß der Erfindung** | | | | |
|---|---|---|---|---|
| 4 a | Omeprazol-Kern + Hilfsstoffe *, ZS.: 3 % E. L 100-55 pH 7,0 msr: 30% E. L 100-55 | 72 | 84 | 89 |
| 4 b | Omeprazol-Kern + Hilfsstoffe*, ZS.: 3 % E. L 100-55 pH 6,0 msr: 30% E. L 100-55 | 40 | 81 | 88 |

| **Vergleichsbeispiel** | | | | |
|---|---|---|---|---|
| 4 c | Omeprazol-Kern + Hilfsstoffe*, keine ZS. msr: 30% E. L 100-55 | 3 | 5 | 8 |
| 4 d | Omeprazol-Kern + Hilfsstoffe*, keine ZS msr: 20% E. L 100-55 | 13 | 37 | 59 |

| | | | | |
|---|---|---|---|---|
| *Hilfsstoffe: Mannit, HPC, Natriumlaurylsulfat ZS: Zwischenschicht E: Eudragit [Gew.-%, bezogen auf den Kern] msr: magensaftresistent(e Schicht) [Gew.-%, bezogen auf den Kern] | | | | |

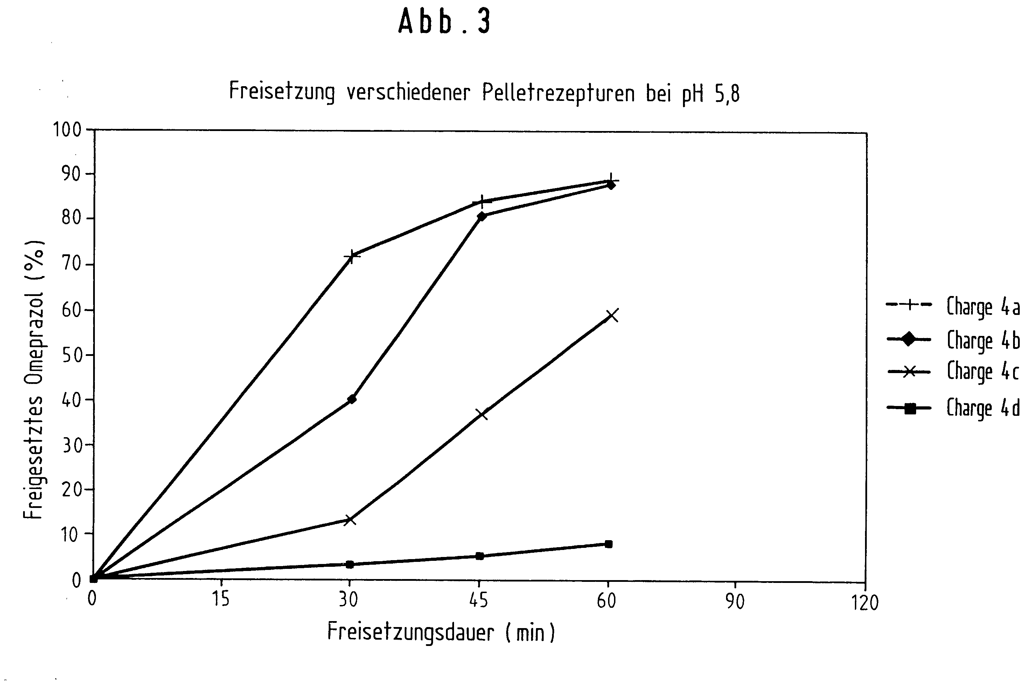

Abbildung 3 zeigt eine graphische Darstellung der Ergebnisse.

Die Dicke der äußeren enterischen Schicht bei Pelletchargen 4 a, b und c ist jeweils gleich (30 Gew.-% bezogen auf den Pelletkern, entspricht ca 40 µm). Trotzdem zeigen überraschend die mit der erfindungsgemäßen, reaktiven Zwischenschicht versehenen Chargen 4 a und 4 b eine deutliche raschere Auflösung im künstlichen Darmsaft, als wenn keine Zwischenschicht vorhanden ist (Charge 4 c). Dies ist auch dann noch der Fall, wenn die enterische Überzugsschicht noch dünner ausgebildet ist (Charge 4 d, 20 Gew.-% bezogen auf den Pelletkern, entspricht ca 30 µm). Dies erlaubt bei den erfindungsgemäßen Arzneiformen die Dicke der äußeren enterischen Schicht weiter zu erhöhen, was eine verbesserte Arzneimittelsicherheit gegenüber bekannten Präparaten bietet, ohne daß das Freisetungsverhalten - wie an sich zu erwarten wäre - negativ beeinflußt wird.

### Beispiel 5

### Verbesserte Stabilität des wirkstoffhaltigen Kerns

Es wurden in der Reibschale Granulate aus Omeprazol mit verschiedenen Hilfsstoffen hergestellt. Nach offener Lagerung über 32 Tage bei 40°C und 75% relativer Feuchte wurde mit HPLC der Restgehalt Omeprazol sowie das Auftreten von Degradationsprodukt bestimmt. Als Hilfsstoffe wurden im alkalischen Milieu pufferndes Na₂HPO₄, Texapon, Lactose, L-HPC, mikrokristalline Cellulose und Mannit (Charge 5 b), sowie eine Kombination dieser Hilfsstoffe ohne Na₂HPO₄ (Charge 5 c) eingesetzt. Diese Chargen wurden mit einem Omeprazolgranulat verglichen, das neben dem Wirkstoff als Hilfsstoffe nur HPC und Mannit (Charge 5 a) enthielt. Die Ergebnisse sind in Tabelle 5 wiedergegeben.

**Tabelle 5**

| **Charge** | **Rezeptur** (Granulat) | **Gehalt Omeprazol** [Gew.-%] | **Degradationsprodukt von Omeprazol** [Flächen-%] |
|---|---|---|---|
| | Lagerungsbedingungen | 40°C / 75 % rel. Feuchte | |
| | Lagerungsdauer | nach Herst. 32 Tage | nach Herst. 32 Tage |

| **erfindungsgemäß bevorzugt** | | | |
|---|---|---|---|
| 5 a | Omeprazol Mannit HPC | 100 ⇒ 100 | < 0,1 ⇒ < 0,5 |

| **Referenzbeispiel** | | | |
|---|---|---|---|
| 5 b | Omeprazol Hilfsstoffe Na₂ HPO₄ | 100 ⇒ 100 | < 0,1 ⇒ < 0,5 |
| 5 c | Omeprazol Hilfsstoffe ohne Na₂HPO₄ | 100 ⇒ 72 | < 0,1 ⇒ ca.30 |

Erwartungsgemäß zeigt das Omeprazolgranulat ohne alkalisch reagierendem Zusatz (Charge 5 c) eine deutlich verschlechterte Lagerungsstabilität gegenüber einem Omeprazolgranulat mit Na₂HPO₄ als Zusatz (Charge 5 b). So nimmt der Omeprazolgehalt auf 72 Gew.% ab, es entsteht ca. 30 Flächen-% Degradationsprodukt. Überraschenderweise zeigt ein Omeprazolgranulat mit Mannit und Hydroxypropylcellulose als einzige Hilfsstoffe, insbesondere ohne alkalisch reagierende Zusätze (Charge 5 a) ebenfalls eine hervorragende Lagerungsstabilität. In der erfindungsgemäß bevorzugten Arzneiform ist es daher nicht notwendig und auch nicht bevorzugt, alkalische reagierende Hilfsstoffe oder Omeprazolsalz im Kern zu verwenden, da gegebenenfalls aus dem Kern in die reaktive Zwischenschicht eindiffundierende alkalische Substanzen den "Self-Repair-Mechanismus", wie vorstehend erläutert, behindern können.

### Beispiel 6

### Herstellung von erfindungsgemäßen Arzneiformen

### Rezepturbeispiele

### Arzneiform A

| Kern: | |
|---|---|
| Omeprazol | 210,00 g |
| Mannit | 781,60 g |
| Hydroxypropylcellulose | 3,30 g |
| Natriumlaurylsulfat | 5,00 g |

| Zwischenschicht: | |
|---|---|
| Eudragit® L100-55 mit | |
| NaOH auf pH 7,0 neutralisiert | 50,00 g |
| Triethylcitrat | 5,00 g |

| Magensaftresistente (Außen)Schicht: | |
|---|---|
| Eudragit® L100-55 | 300,00 g |
| Triethylcitrat | 30,00 g |
| Talkum mikronisiert | 150,00 g |

### Arzneiform B

| Kern: | |
|---|---|
| Omeprazol | 210,00 g |
| Mannit | 781,60 g |
| Hydroxypropylcellulose | 3,30 g |
| Natriumlaurylsulfat | 5,00 g |
| Zwischenschicht: | |
| Eudragit® L 100-55 mit | |
| NaOH auf pH 5,5 neutralisiert | 50,00 g |
| Triethylcitrat | 5,00 g |
| Talkum | 15,00 g |

| Magenssaftresistente (Außen)Schicht: | |
|---|---|
| Eudragit® L100-55 | 400,00 g |
| Triethylcitrat | 40,00 g |
| Talkum mikronisiert | 200,00 g |

Die vorgewogenen Komponenten Omeprazol, Mannit und Natriumlaurylsulfat werden in einem Mischer gegeben und vermischt. Eine Granulierungsflüssigkeit aus in Isopropanol gelöster Hydroxypropylcellulose wird langsam zu den vorgemischten Komponenten im Mischer unter konstantem Rühren zugegeben. Falls erforderlich, kann für eine bessere Pelletausbildung weiteres Isopropanol zugegeben werden. Die Mischzeit beträgt 10 bis 20 min, bis die Mehrzahl der Pellets eine erwünschte mittlere Größe von ca. 1000 µm haben.

Die feuchten Pellets werden in einem Trockner bei ca. 60°C für ca. 40 Min. getrocknet. Pellets mit einem Durchmesser von < 700 µm oder > 1200 µm werden ausgesiebt.

Die Pellets werden in einem fluidisierten Zustand gehalten, während zunächst eine Überzugsdispersion I und anschließend eine Überzugsdispersion II auf die Pellets mit einer konstanten Rate aufgesprüht wird.

Zur Herstellung der Überzugsdispersion I wird gereinigtes Wasser in ein Edelstahlgefäß gefüllt und Natriumhydroxid im Wasser aufgelöst. Die Natriumhydroxidlösung wird unter Rühren zum mikronisierten Talkum gegeben, dann wird eine Eudragitdispersion langsam zu der Natriumhydroxid/Talkumdispersion unter Rühren zugegeben, wobei Klumpen und Schaumbildung vermieden werden müssen. Nach Zugabe von Triethylcitrat zur Dispersion wird das Rühren für mindestens 15 min fortgesetzt, wobei hierbei der pH-Wert auf pH 7,0 bzw. pH 5,5 mit Natriumhydroxidlösung eingestellt wird. Während der Ausbildung des Überzugs muß die Dispersion kontinuierlich weitergerührt werden.

Zur Herstellung der Überzugsdispersion II wird mikronisiertes Talkum in gereinigtem Wasser dispergiert. Anschließend wird die so erhaltene wässrige Dispersion zur Eudragitdispersion unter Rühren zugesetzt, wobei Auftreten von Klumpen oder Schaum vermieden werden muß. Nach Zugabe von Triethylcitrat wird die Dispersion für mindestens weitere 15 min gerührt. Die Omeprazolpellets werden dann in eine Beschichtungsapparatur überführt und bei 30°C zunächst mit der Überzugsdispersion I und dann mit der Überzugsdispersion II befilmt. Die fertigen Omeprazolpellets werden in Hartgelatinekapseln gefüllt.

### Beispiel 7

*In vitro* Versuche zur chemischen Stabilität der erfindungsgemäßen Arzneiform:

Es ist bekannt, daß Omeprazol bei längerer Lagerung an Wirksamkeit verliert, was auf einen Abbau der Wirksubstanz zurückzuführen ist. Durch Aufbringen geeigneter Schutzschichten kann dieser chemische Abbau von Omeprazol auf ein Minimum reduziert werden.

In Stabilitätsversuchen unter Streßbedingungen (40°C / 75 % rel. Feuchte) konnte gezeigt werden, daß die erfindungsgemäße Arzneiform bei Lagerung in verschlossenen braunen Schraubdeckelgläsern für 4 Wochen weniger als 2 Gew.-% Wirkstoff verliert (Mittelwerte von je drei Gehaltsbestimmungen). Ein unter identischen Bedingungen gelagertes Handelsprodukt verlor dagegen im gleichen Zeitraum mehr als 80 Gew.-% Wirkstoff (siehe Tabelle 6).

**Tabelle 6**

| **Arzneiform (Charge)** | **Omeprazolgehalt** nach 12 Wochen Lagerung bei 40°C/75% r.F. im verschlossenen Schraubdeckelglas [Gew.-%] |
|---|---|
| **gemäß der Erfindung** | |
| 6 a | 98,9 |
| 6 b | 98,3 |
| 6 c | 99,3 |

| **Handelsprodukt** | |
|---|---|
| 6 d | 16,2 |

In weiteren Stabilitätsversuchen unter Langzeit- und Streßbedingungen (25°C / 60 % r.F.; 30°C / 60 % r.F.; 40°C / 75 % r.F.) konnte gezeigt werden, daß die erfindungsgemäße Arzneiform bei Lagerung im Primärpackmittel für 12 Wochen weniger als 5 Gew.-% Wirkstoff verliert (Mittelwerte von je drei Gehaltsbestimmungen). Die Ergebnisse sind in Tabelle 7 dargestellt.

**Tabelle 7**

| **Arzneiform (Charge)** | **Omeprazolgehalt** nach 12 Wochen Lagerung [Gew.-%] | | |
|---|---|---|---|
| Lagerungsbedingungen | 25°C/60% r.F. | 30°C/60% r.F. | 40°C/75% r.F. |
| 7 a | 98,9 | 97,9 | 97,8 |
| 7 b | 99,1 | 97,8 | 95,3 |
| 7 c | 100,5 | 103,3 | 98,0 |

Die Ergebnisse zeigen deutlich, daß bei der erfindungsgemäßen Arzneiform keine signifikante Gehaltsabnahme vom Ausgangswert festzustellen war.

### Beispiel 8

### Bestimmung der Magensaftresistenz

Zur Bestimmung der Magensaftresistenz wurden Proben der erfindungsgemäßen Arzneiform gemäß Beispiel 6 einem *in vitro* Test unterzogen. Dabei wurde die jeweilige Probe, die sich in einem Basket befand, bei pH 1,2 und einer Temperatur von 37°C sowie 100 upm des Basket in dem sauren Medium für 120 min belassen und anschließend die Probe auf den verbleibenden Wirkstoffgehalt analysiert. Die dabei erhaltenen Werte sind in Tabelle 8 zusammengefaßt. Sämtliche Werte zeigen, daß sich unter den gewählten Bedingungen kein signifikanter Abbau zum Ausgangswert ergibt.

**Tabelle 8**

| **Arzneiform (Charge)** | **Omeprazolrestgehalt** [Gew.-%] |
|---|---|
| **gemäß der Erfindung** | |
| 8 a | 98,4 |
| 8 b | 98,9 |
| 8 c | 96,0 |

### Beispiel 9

*In vitro* Untersuchungen zur Wirkstoff-Freisetzung

Für die folgenden Versuche wurde ebenfalls eine erfindungsgemäße Arzneiform gemäß Beispiel 6 untersucht. Die jeweilige Probe, die sich in einem Basket befand, wurde 120 Minuten lang bei 37°C einem Medium (1000 ml) von pH 1,2 ausgesetzt.

Nach der vorstehend genannten Verweilzeit in saurem Medium wurde dieses durch ein alkalisches Medium (pH 6,8, phosphatgepuffert) ersetzt, und die Probe jeweils für einen Zeitraum von 5, 10, 15, 20, 30, 60 Minuten darin belassen. Nach den genannten Zeitintervallen wurden Analysen zum freigesetzten Wirkstoff durchgeführt.

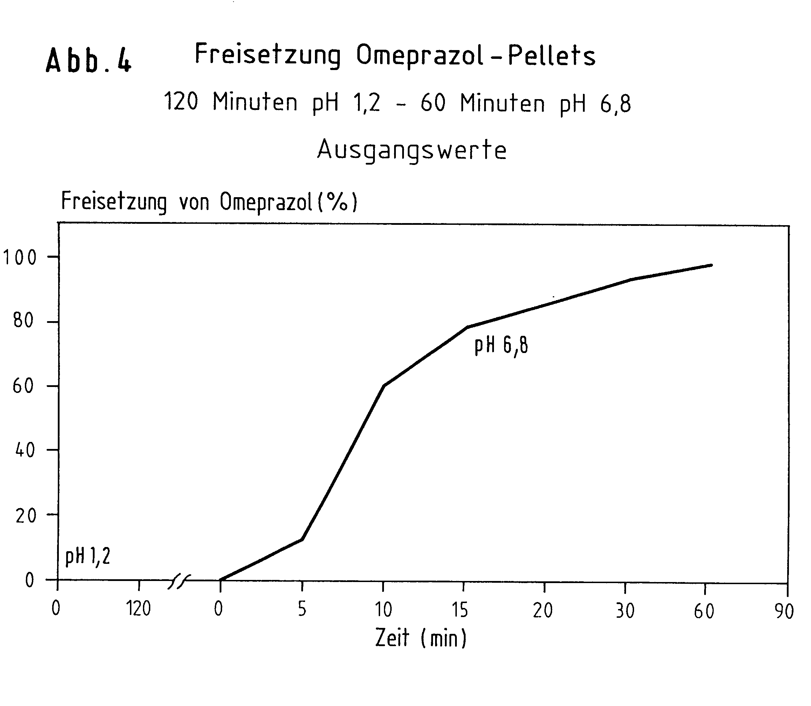

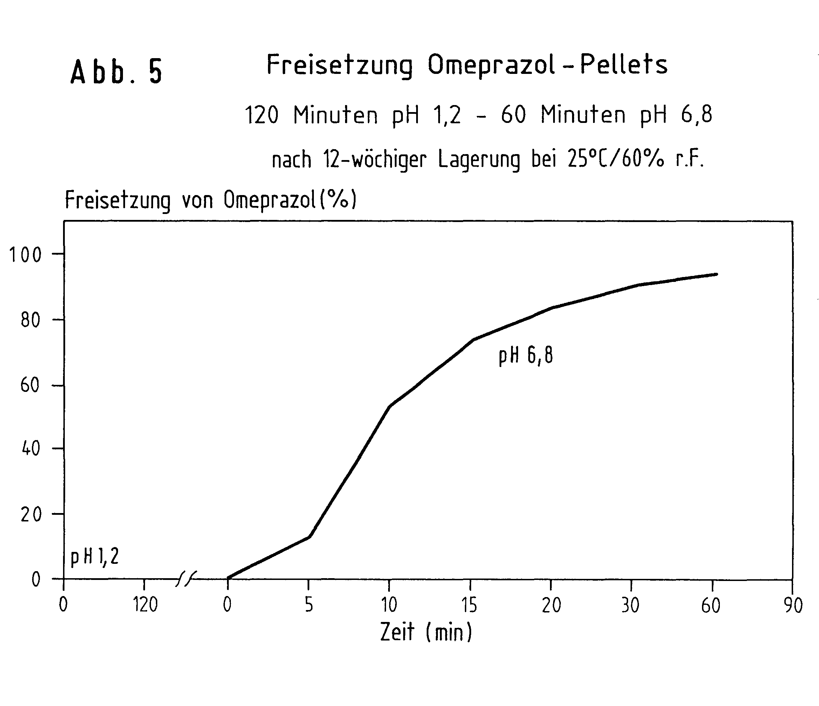

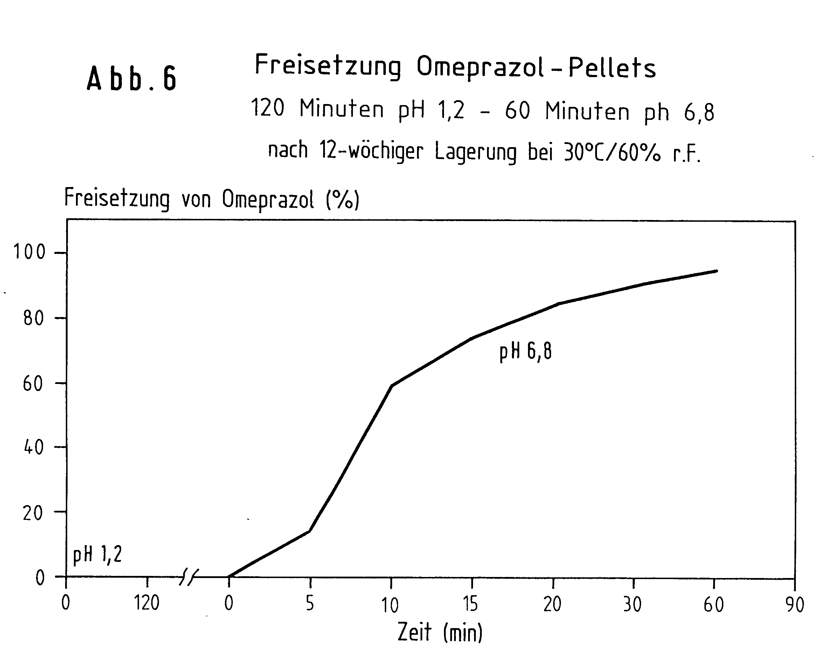

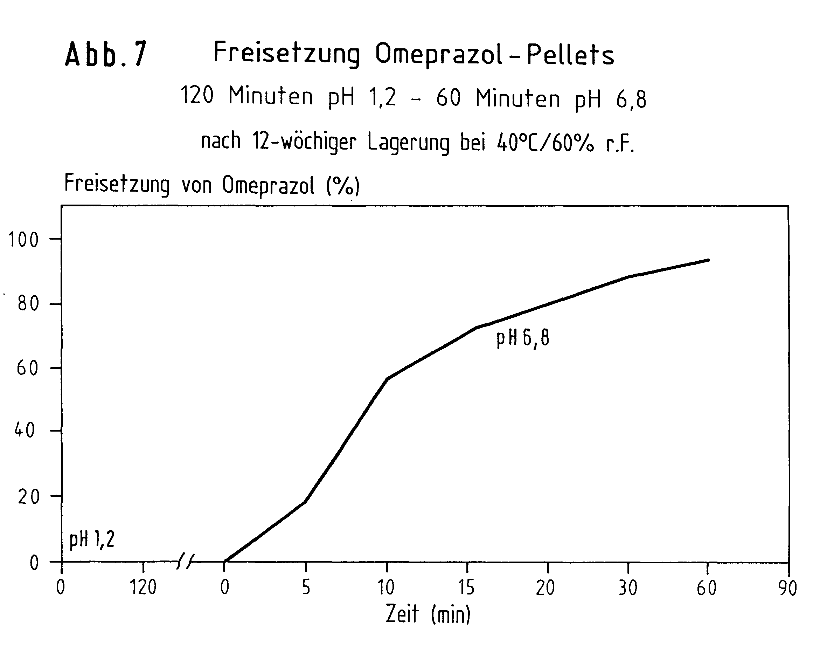

Die Bestimmung der in vitro Wirkstoff-Freisetzung erfolgte an Proben vor ihrer Einlagerung (Abbildung 4) und nach einer zwölfwöchigen Lagerung bei 25°C/60% r.F. (Abbildung 5), bei 30°C/60% r.F. (Abbildung 6) und 40°C/75% r.F. (Abbildung 7). Die Arzneimittel wurden zuvor in das vorgesehene Packmittel abgefüllt. Die erhaltenen Werte sind für die jeweiligen erfindungsgemäßen Chargen in den Abbildungen 4 bis 7 dargestellt, und zeigen, daß über den Lagerzeitraum die Freisetzung stabil ist.

### Beispiel 10

### Kombinationspräparat Omeprazol und Diclofenac

### Kapselformulierung C:

Eine Kapsel enthält 210 mg Diclofenac-Pellets entsprechend 75 mg Diclofenac-Na und 160 mg Omeprazol-Pellets entsprechend 20 mg Omeprazol. Sowohl die Diclofenac-Pellets als auch die Omeprazol-Pellets wurden nach dem Herstellungsverfahren gemäß Beispiel 6 hergestellt.

### Kapselformulierung D:

Eine Kapsel enthält 420 mg Diclofenac-Pellets entsprechend 150 mg Diclofenac-Na und 160 mg Omeprazol-Pellets entsprechend 20 mg Omeprazol, die gemäß Beispiel 6 hergestellt wurden. Die Diclofenac-Pellets wurden nach dem in EP 0 348 808 wiedegegebenen Verfahren hergestellt.

## Patentansprüche

1. Stabile Arzneiform zur oralen Verabreichung, welche
(a) einen Kern, der einen Wirkstoff, ausgewählt aus Omeprazol, Lansoprazol und Pantoprazol, zusammen mit üblichen pharmazeutischen Hilfsstoffen enthält,
(b) eine auf den Kern aufgebrachte Zwischenschicht, und
(c) eine magensaftresistente Außenschicht umfaßt,
**dadurch gekennzeichnet, daß**
in (b) eine reaktive Zwischenschicht aus einem mit Alkalien teilneutralisierten magensaftresistenten Polymer-Schichtmaterial mit Kationenaustauscher-kapazität vorliegt.

2. Arzneiform nach Anspruch 1, **dadurch gekennzeichnet, daß** die Alkalien ausgewählt sind aus Natriumhydroxid und Kaliumhydroxid.

3. Arzneiform nach Anspruch 1 oder 2, **dadurch gekennzeichnet, daß** der pharmazeutische Hilfsstoff ausgewählt ist aus Mannit und Hydroxypropylcellulose.

4. Arzneiform nach Anspruch 1 bis 3, **dadurch gekennzeichnet, daß** der Kern zusätzlich ein Netzmittel umfaßt.

5. Arzneiform nach Anspruch 4, **dadurch gekennzeichnet, daß** das Netzmittel ausgewählt ist aus Natriumlaurylsulfat, Sorbitanfettsäureester und Polyoxyethylensorbitan-Fettsäureester.

6. Arzneiform nach Anspruch 1 bis 5, **dadurch gekennzeichnet, daß** der Kern in Form von Pelletkernen, Tabletten, Mikrotabletten oder als Granulat vorliegt.

7. Arzneiform nach Anspruch 1 bis 6, **dadurch gekennzeichnet, daß** das magensaftresistente Polymer-Schichtmaterial in der reaktiven Zwischenschicht auf einen pH-Bereich von 5,5 bis 7,0, bevorzugt 5,5 bis 6,5, teilneutralisiert ist.

8. Arzneiform nach Anspruch 7, **dadurch gekennzeichnet, daß** das teilneutralisierte magensaft-resistente Polymer-Schichtmaterial ausgewählt ist aus teilneutralisiertem Eudragit® L100-55, Eudragit® L100, Hydroxypropylmethylcellulosephthalat (HPMCP) und Celluloseacetatphthalat (CAP).

9. Arzneiform nach Anspruch 1 bis 8, **dadurch gekennzeichnet, daß** die reaktive Zwischenschicht zusätzlich einen Weichmacher umfaßt.

10. Arzneiform nach Anspruch 9, **dadurch gekennzeichnet, daß** der Weichmacher ausgewählt ist aus Triethylcitrat, Acetyltriethylcitrat, acetylierten Monoglyceriden, Propylenglykol und Polyethylenglykolen.

11. Arzneiform nach Anspruch 1 bis 10, **dadurch gekennzeichnet, daß** die reaktive Zwischenschicht beim Eindringen von Protonen durch die Außenschicht eine gelartige Schicht ausbildet.

12. Arzneiform nach Anspruch 1 bis 11, **dadurch gekennzeichnet, daß** die reaktive Zwischenschicht eine Dicke von 5 bis 30µm besitzt.

13. Arzneiform nach Anspruch 1 bis 12, **dadurch gekennzeichnet, daß** die magensaftresistente Außenschicht in (c) Eudragit® L100-55, Eudragit® L100, Hydroxypropylmethylcellulosephthalat (HPMCP) und/oder Celluloseacetatphthalat (CAP) enthält.

14. Arzneiform gemäß Anspruch 13, **dadurch gekennzeichnet, daß** die magensaftresistente Außenschicht pharmazeutisch verträgliche Antihaftmittel, Dispergierungsmittel, Pigmente und/oder Farbstoffe enthält.

15. Arzneiform nach Anspruch 14, **dadurch gekennzeichnet, daß** das Antihaftmittel Talkum ist.

16. Arzneiform nach Anspruch 1 bis 15, **dadurch gekennzeichnet, daß** die magensaftresistente Außenschicht eine Schichtdicke von 20 bis 60 µm, bevorzugt 30 bis 60 µm aufweist.

17. Arzneiform nach Anspruch 1 bis 16, welche
(a) einen Kern, der einen Wirkstoff, ausgewählt aus Omeprazol, Lansoprazol und Pantoprazol, zusammen mit Mannit und Hydroxypropylcellulose als Hilfsstoffe ohne alkalische Zusätze enthält,
(b) eine auf den Kern aufgebrachte reaktive Zwischenschicht mit einer Dicke von 5 bis 30 µm aus mit Natriumhydroxid auf einen pH-Bereich von 5,5 bis 7,0 teilneutralisiertem Eudragit® L100-55, und
(c) eine magensaftresistente Außenschicht mit einer Dicke von 30 bis 60 µm aus Eudragit® L100-55 umfaßt.

18. Arzneiform nach Anspruch 1 bis 17, **dadurch gekennzeichnet, daß** die reaktive Zwischenschicht als eine Vielzahl von Einzelschichten ausgebildet ist.

19. Arzneiform nach Anspruch 1 bis 18, **dadurch gekennzeichnet, daß** die magensaftresistente Außenschicht als eine Vielzahl von Einzelschichten ausgebildet ist.

20. Arzneiform nach Anspruch 1 bis 19, **dadurch gekennzeichnet, daß** der pH-Übergang an der Grenze von der magensaftresistenten Außenschicht zur reaktiven Zwischenschicht als Gradient ausgebildet ist.

21. Verfahren zur Herstellung einer stabilen Arzneiform zur oralen Verabreichung nach einem der Ansprüche 1 bis 20, **dadurch gekennzeichnet, daß** man
(a) als Kern einen Formling bildet, der einen Wirkstoff, ausgewählt aus Omeprazol, Lansoprazol und Pantoprazol, zusammen mit üblichen pharmazeutischen Hilfsstoffen enthält
(b) auf die Formlinge eine Zwischenschicht aufbringt, und
(c) die so überzogenen Formlinge mit einer magensaftresistenten Schicht befilmt,
**dadurch gekennzeichnet, daß** man
in (b) eine reaktive Zwischenschicht aus einem mit Alkalien teilneutralisierten magensaftresistenten Polymer-Schichtmaterial mit Kationenaustauscher-kapazität aufbringt.

22. Verfahren nach Anspruch 21, **dadurch gekennzeichnet, daß** man das magensaftresistente Polymer-Schichtmaterial vor dem Aufsprühen auf den Kern mit Alkalien auf einen pH-Bereich von 5,5 bis 7,0 teilneutralisiert.

23. Verfahren nach Anspruch 22, **dadurch gekennzeichnet, daß** man als Alkalien Natriumhydroxid oder Kaliumhydroxid verwendet.

24. Verfahren nach Anspruch 21, **dadurch gekennzeichnet, daß** man in Schritt (a) Isopropanol als ein Lösungsmittel verwendet.

25. Pharmazeutische Zusammensetzung, welche zusätzlich zu einer stabilen Arzneiform nach Anspruch 1 bis 20 Diclofenac als weiteren Wirkstoff enthält.

26. Pharmazeutische Zusammensetzung nach Anspruch 25, **dadurch gekennzeichnet, daß** das Diclofenac als Formulierung vorliegt, welche
(a) einen Diclofenac-haltigen Kern zusammen üblichen Hilfsstoffen,
(b) eine auf den Kern aufgebrachte reaktive Zwischenschicht aus mit Alkalien teilneutralisiertem magensaftresistenten Polymer-Schichtmaterial, und
(c) eine magensaftresistente Außenschicht
umfaßt.

27. Pharmazeutische Zusammensetzung nach Anspruch 25, **dadurch gekennzeichnet, daß** das Diclofenac als Pelletformulierung umfassend eine Mischung von magensaftresistent umhüllten Pellets und retardiert durchlässigen Pellets vorliegt.

28. Pharmazeutische Kapselformulierung, **dadurch gekennzeichnet, daß** sie eine stabile Arzneiform nach Anspruch 1 bis 20 oder eine Zusammensetzung nach Anspruch 25 bis 27 als Pellets enthält.

## Claims

1. Stable drug form for oral administration, which comprises
(a) a core which contains an active ingredient selected from omeprazole, lansoprazole and pantoprazole, together with conventional pharmaceutical excipients,
(b) an intermediate layer applied to the core, and
(c) an external layer which is resistant to gastric juice,
**characterized in that** a reactive intermediate layer composed of a polymeric layer material which is resistant to gastric juice, is partially neutralized with alkalis and has cation exchange capacity is present in (b).

2. Drug form according to Claim 1, **characterized in that** the alkalis are selected from sodium hydroxide and potassium hydroxide.

3. Drug form according to Claim 1 or 2, **characterized in that** the pharmaceutical excipient is selected from mannitol and hydroxypropylcellulose.

4. Drug form according to Claim 1 to 3, **characterized in that** the core additionally comprises a wetting agent.

5. Drug form according to Claim 4, **characterized in that** the wetting agent is selected from sodium lauryl sulphate, sorbitan fatty acid ester and polyoxyethylenesorbitan fatty acid ester.

6. Drug form according to Claim 1 to 5, **characterized in that** the core is in the form of pellet cores, tablets, microtablets or as granules.

7. Drug form according to Claim 1 to 6, **characterized in that** the polymeric layer material which is resistant to gastric juice in the reactive intermediate layer is partially neutralized to a pH range from 5.5 to 7.0, preferably 5.5 to 6.5.

8. Drug form according to Claim 7, **characterized in that** the partially neutralized polymeric layer material which is resistant to gastric juice is selected from partially neutralized Eudragit® L100-55, Eudragit® L100, hydroxypropylmethylcellulose phthalate (HPMCP) and cellulose acetate phthalate (CAP).

9. Drug form according to Claim 1 to 8, **characterized in that** the reactive intermediate layer additionally comprises a plasticizer.

10. Drug form according to Claim 9, **characterized in that** the plasticizer is selected from triethyl citrate, acetyl triethyl citrate, acetylated monoglycerides, propylene glycol and polyethylene glycols.

11. Drug form according to Claim 1 to 10, **characterized in that** the reactive intermediate layer forms a gelatinous layer when protons penetrate in through the external layer.

12. Drug form according to Claim 1 to 11, **characterized in that** the reactive intermediate layer has a thickness of from 5 to 30 µm.

13. Drug form according to Claim 1 to 12, **characterized in that** the external layer which is resistant to gastric juice in (c) contains Eudragit® L100-55, Eudragit® L100, hydroxypropylmethylcellulose phthalate (HPMCP) and/or cellulose acetate phthalate (CAP).

14. Drug form according to Claim 13, **characterized in that** the external layer which is resistant to gastric juice contains pharmaceutically acceptable antiadhesion agents, dispersants, pigments and/or colours.

15. Drug form according to Claim 14, **characterized in that** the antiadhesion agent is talc.

16. Drug form according to Claim 1 to 15, **characterized in that** the external layer which is resistant to gastric juice has a layer thickness of from 20 to 60 µm, preferably 30 to 60 µm.

17. Drug form according to Claim 1 to 16, which comprises
(a) a core which contains an active ingredient selected from omeprazole, lansoprazole and pantoprazole, together with mannitol and hydroxypropylcellulose as excipients without alkaline additions,
(b) a reactive intermediate layer which is applied to the core and has a thickness of from 5 to 30 µm and is composed of Eudragit® L100-55 which is partially neutralized with sodium hydroxide to a pH range from 5.5 to 7.0, and
(c) an external layer which is resistant to gastric juice and has a thickness of from 30 to 60 µm and is composed of Eudragit® L100-55.

18. Drug form according to Claim 1 to 17, **characterized in that** the reactive intermediate layer is designed as a plurality of individual layers.

19. Drug form according to Claim 1 to 18, **characterized in that** the external layer which is resistant to gastric juice is designed as a plurality of individual layers.

20. Drug form according to Claim 1 to 19, **characterized in that** the pH change at the boundary between the external layer which is resistant to gastric juice and the reactive intermediate layer is designed as gradient.

21. Process for producing a stable drug form for oral administration according to any of Claims 1 to 20, **characterized in that**
(a) a shaped article which contains an active ingredient selected from omeprazole, lansoprazole and pantoprazole, together with conventional pharmaceutical excipients, is formed as core,
(b) an intermediate layer is applied to the shaped article, and
(c) the shaped article coated in this way is covered with a layer which is resistant to gastric juice,
**characterized in that** a reactive intermediate layer composed of a polymeric layer material which is resistant to gastric juice, is partially neutralized with alkalis and has cation exchange capacity is applied in (b).

22. Process according to Claim 21, **characterized in that** the polymeric layer material which is resistant to gastric juice is partially neutralized before spraying onto the core with alkalis to a pH range from 5.5 to 7.0.

23. Process according to Claim 22, **characterized in that** sodium hydroxide or potassium hydroxide are used as alkalis.

24. Process according to Claim 21, **characterized in that** isopropanol is used as a solvent in step (a).

25. Pharmaceutical composition which, in addition to a stable drug form according to Claim 1 to 20, contains diclofenac as further active ingredient.

26. Pharmaceutical composition according to Claim 25, **characterized in that** the diclofenac is present as formulation which comprises
(a) a diclofenac-containing core together [lacuna] conventional excipients
(b) a reactive intermediate layer which is composed of polymeric layer material which is resistant to gastric juice and is partially neutralized with alkalis and which is applied to the core, and
(c) an external layer which is resistant to gastric juice.

27. Pharmaceutical composition according to Claim 25, **characterized in that** the diclofenac is present as pellet formulation comprising a mixture of pellets with a covering which is resistant to gastric juice and pellets with delayed permeability.

28. Pharmaceutical capsule formulation, **characterized in that** it contains a stable drug form according to Claim 1 to 20 or a composition according to Claim 25 to 27 as pellets.

## Revendications

1. Forme de médicament stable pour administration orale, comprenant
(a) un noyau constitué d'un agent actif choisi parmi de l'oméprazole, du lansoprazole et du pantoprazole, ainsi que des adjuvants pharmaceutiques usuels,
(b) une couche intermédiaire appliquée sur le noyau, et
(c) une couche extérieure gastro-résistante,
**caractérisée en ce qu'**en (b) est présente une couche intermédiaire réactive constituée d'un matériau de couche polymère gastro-résistant partiellement neutralisé par des alcalis et doté d'une capacité d'échange de cations.

2. Forme de médicament selon la revendication 1, **caractérisée en ce que** les alcalis sont choisis parmi l'hydroxyde de sodium et l'hydroxyde de potassium.

3. Forme de médicament selon la revendication 1 ou 2, **caractérisée en ce que** l'adjuvant pharmaceutique est choisi parmi le mannitol et l'hydroxypropylcellulose.

4. Forme de médicament selon les revendications 1 à 3, **caractérisée en ce que** le noyau comporte en outre un agent mouillant.

5. Forme de médicament selon la revendication 4, **caractérisée en ce que** l'agent mouillant est choisi parmi le laurylsulfate de sodium, des esters d'acide gras de sorbitanne et des esters d'acide gras de polyoxyéthylène sorbitanne.

6. Forme de médicament selon les revendications 1 à 5, **caractérisée en ce que** le noyau se présente sous la forme de noyaux de comprimés, de tablettes, de microtablettes ou de granulés.

7. Forme de médicament selon les revendications 1 à 6, **caractérisée en ce que** le matériau de couche polymère gastro-résistant dans la couche intermédiaire réactive est partiellement neutralisé dans une plage de pH de 5,5 à 7,0, de préférence de 5,5 à 6,5.

8. Forme de médicament selon la revendication 7, **caractérisée en ce que** le matériau de couche polymère gastro-résistant et partiellement neutralisé est choisi parmi de l'Eudragit® L100-55, de l'Eudragit® L100, du phtalate d'hydroxypropylméthylcellulose (HPMCP) et de l'acétophtalate de cellulose (CAP) partiellement neutralisé.

9. Forme de médicament selon les revendications 1 à 8, **caractérisée en ce que** la couche intermédiaire réactive comporte en outre un plastifiant.

10. Forme de médicament selon la revendication 9, **caractérisée en ce que** le plastifiant est choisi parmi le citrate de triéthyle, le citrate d'acétyltriéthyle, des monoglycérides acétylés, du propylène glycol et des polyéthylène glycols.

11. Forme de médicament selon les revendications 1 à 10, **caractérisée en ce que** la couche intermédiaire réactive forme une couche gélatineuse lorsque des protons pénètrent la couche extérieure.

12. Forme de médicament selon les revendications 1 à 11, **caractérisée en ce que** la couche intermédiaire réactive possède une épaisseur de 5 à 30 µm.

13. Forme de médicament selon les revendications 1 à 12, **caractérisée en ce que** la couche extérieure gastro-résistante dans (c) contient de l'Eudragit® L100-55, de l'Eudragit® L100, du phtalate d'hydroxypropylméthylcellulose (HPMCP) et/ou de l'acétophtalate de cellulose (CAP).

14. Forme de médicament selon la revendication 13, **caractérisée en ce que** la couche extérieure gastro-résistante contient des agents antiadhérence, des agents de dispersion, des pigments et/ou des colorants pharmaceutiquement acceptables.

15. Forme de médicament selon la revendication 14, **caractérisée en ce que** l'agent antiadhérence est du talc.

16. Forme de médicament selon les revendications 1 à 15, **caractérisée en ce que** la couche extérieure gastro-résistante possède une épaisseur de couche de 20 à 60 µm, de préférence de 30 à 60 µm.

17. Forme de médicament selon les revendications 1 à 16, comprenant
(a) un noyau constitué d'un agent actif choisi parmi de l'oméprazole, du lansoprazole et du pantoprazole, ainsi que du mannitol et de l'hydroxypropylcellulose en tant qu'adjuvants, sans additifs alcalins,
(b) une couche intermédiaire réactive appliquée sur le noyau, d'une épaisseur de 5 à 30 µm et à base d'Eudragit® L100-55 partiellement neutralisé par de l'hydroxyde de sodium à une plage de pH de 5,5 à 7,0, et
(c) une couche extérieure gastro-résistante, d'une épaisseur de 30 à 60 µm et constituée d'Eudragit® L100-55.

18. Forme de médicament selon les revendications 1 à 17, **caractérisée en ce que** la couche intermédiaire réactive est formée d'une multiplicité de couches individuelles.

19. Forme de médicament selon les revendications 1 à 18, **caractérisée en ce que** la couche extérieure gastro-résistante est formée d'une multiplicité de couches individuelles.

20. Forme de médicament selon les revendications 1 à 19, **caractérisée en ce que** la transition de pH à l'interface entre la couche extérieure gastro-résistante et la couche intermédiaire réactive est réalisée sous forme de gradient.

21. Procédé de préparation d'une forme de médicament stable pour administration orale selon l'une des revendications 1 à 20, **caractérisé en ce que**
(a) on forme comme noyau une ébauche qui contient un agent actif, choisi parmi de l'oméprazole, du lansoprazole et du pantoprazole, ainsi que des adjuvants pharmaceutiques usuels,
(b) on applique sur les ébauches une couche intermédiaire, et
(c) les ébauches ainsi enrobées sont revêtues d'une couche gastro-résistante,
**caractérisé en ce qu'**en (b) on applique une couche intermédiaire réactive constituée d'un matériau de couche polymère gastro-résistant partiellement neutralisé par des alcalis et doté d'une capacité d'échange de cations.

22. Procédé selon la revendication 21, **caractérisé en ce que** l'on neutralise partiellement le matériau de couche polymère gastro-résistant, avant la pulvérisation sur le noyau, au moyen d'alcalis et à une plage de pH de 5,5 à 7,0.

23. Procédé selon la revendication 22, **caractérisé en ce que** l'on utilise comme alcalis de l'hydroxyde de sodium ou de l'hydroxyde de potassium.

24. Procédé selon la revendication 21, **caractérisé en ce que**, dans l'étape (a), on utilise de l'isopropanol en tant qu'un solvant.

25. Composition pharmaceutique contenant, outre une forme de médicament stable selon les revendications 1 à 20, du diclofénac comme autre agent actif.

26. Composition pharmaceutique selon la revendication 25, **caractérisée en ce que** le diclofénac se trouve en tant que formulation qui comprend
(a) un noyau contenant du diclofénac et des additifs usuels,
(b) une couche intermédiaire réactive appliquée sur le noyau et constituée d'un matériau de couche polymère gastro-résistant et partiellement neutralisé par des alcalis, et
(c) une couche extérieure gastro-résistante.

27. Composition pharmaceutique selon la revendication 25, **caractérisée en ce que** le diclofénac se trouve en tant que formulation de comprimé comprenant un mélange de comprimés enrobés gastro-résistants et de comprimés à perméabilité retardée.

28. Formulation pharmaceutique de capsules, **caractérisée en ce qu'**elle contient une forme de médicament stable selon les revendications 1 à 20 ou une composition selon les revendications 25 à 27 sous forme de comprimés.
